(19) Europäisches Patentamt
European Patent Office
Office européen des brevets

(11) Veröffentlichungsnummer: **0 057 660**
**B1**

(12) # EUROPÄISCHE PATENTSCHRIFT

(45) Veröffentlichungstag der Patentschrift: **21.08.85**

(21) Anmeldenummer: **82730008.8**

(22) Anmeldetag: **01.02.82**

(51) Int. Cl.⁴: **C 07 C 59/46,** C 07 C 103/19, C 07 C 125/065, C 07 C 177/00, A 61 K 31/16, A 61 K 31/215 // A61K31/557

(54) **Neue Prostacyclin-Derivate, Verfahren zu ihrer Herstellung sowie ihre Verwendung als Arzneimittel.**

(30) Priorität: **02.02.81 DE 3104044**

(43) Veröffentlichungstag der Anmeldung: **11.08.82 Patentblatt 82/32**

(45) Bekanntmachung des Hinweises auf die Patenterteilung: **21.08.85 Patentblatt 85/34**

(84) Benannte Vertragsstaaten: **AT BE CH DE FR GB IT LI LU NL SE**

(56) Entgegenhaltungen:
**EP - A - 0 011 591**
**GB - A - 2 014 143**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Skuballa, Werner, Dr., Olwenstrasse 25, D-1000 Berlin 28 (DE)**
Erfinder: **Radüchel, Bernd, Dr., Gollanczstrasse 132, D-1000 Berlin 28 (DE)**
Erfinder: **Schwarz, Norbert, Dr., Hauptstrasse 118, D-1000 Berlin 62 (DE)**
Erfinder: **Vorbrüggen, Helmut, Prof.Dr., Wilkestrasse 7, D-1000 Berlin 27 (DE)**
Erfinder: **Casals, Jorge, Dr., Wichernstrasse 20, D-1000 Berlin 33 (DE)**
Erfinder: **Schillinger, Ekkehard, Dr., Im Amseltal 50, D-1000 Berlin 28 (DE)**
Erfinder: **Town, Michael Harold, Dr., Buchsbaumweg 3, D-1000 Berlin 47 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 057 660 B1

**Beschreibung**

Die Erfindung betrifft neue Prostacyclin-Derivate, Verfahren zu ihrer Herstellung sowie ihre Verwendung als Arzneimittel.

In den deutschen Offenlegungsschriften DE-OS 2 845 770, 2 900 352, 2 902 442, 2 909 088 und 2 912 409 werden (5E)- und (5Z)-6a-Carbaprostaglandin-$I_2$-Analoga beschrieben. In DE-OS 2 904 655 werden $\Delta^2 PGJ_2$-Derivate mit einer Doppelbindung in 18-Stellung beschrieben. Die Nomenklatur der erfindungsgemäßen Verbindungen basiert auf einem Vorschlag von Morton und Brokaw (J. Org. Chem. 44, 2880 [1979]). Bei der Synthese dieser Verbindungen entstehen stets zwei Doppelbindungsisomere, die durch den Zusatz (5E) oder (5Z) charakterisiert werden. Die beiden Isomeren dieses Prototyps werden durch folgende Strukturformeln verdeutlicht:

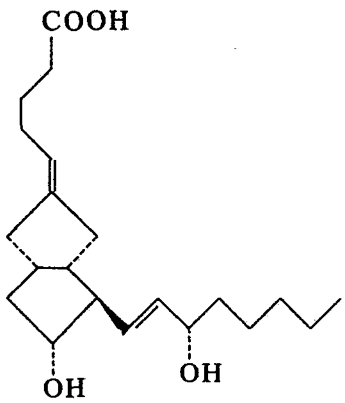

(5E)-6a-Carbaprostaglandin-$I_2$

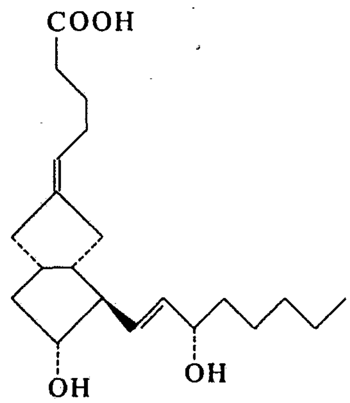

(5Z)-6a-Carbaprostaglandin-$I_2$

Aus dem sehr umfangreichen Stand der Technik der Prostacycline und ihrer Analoga weiß man, daß diese Stoffklasse auf Grund ihrer biologischen und pharmakologischen Eigenschaften zur Behandlung von Säugetieren, einschließlich Menschen geeignet ist. Ihre Verwendung als Arzneimittel stößt jedoch häufig auf Schwierigkeiten, da sie eine für therapeutische Zwecke zu kurze Wirkungsdauer besitzen. Alle Strukturveränderungen haben das Ziel, die Wirkungsdauer sowie die Selektivität der Wirksamkeit zu steigern.

Es wurde nun gefunden, daß durch Einführung einer Doppelbindung in die 2,3-Position des Carbacyclins eine längere Wirkungsdauer, größere Selektivität und bessere Wirksamkeit erzielt werden kann. Die erfindungsgemäßen Verbindungen wirken blutdrucksenkend und bronchodilatatorisch. Sie sind außerdem zur Inhibierung der Thrombozytenaggregation, der Vasodilatation und der Inhibierung der Magensäuresekretion geeignet.

Die Erfindung betrifft Carbacyclinderivate der allgemeinen Formel I

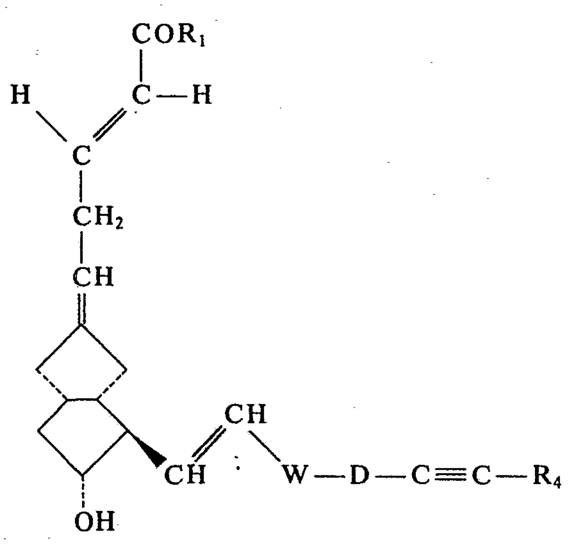

(I)

worin

$R_1$ den Rest $OR_2$, wobei $R_2$ Wasserstoff oder Alkyl mit 1–4 C-Atomen bedeuten kann, oder den Rest $NHR_3$ mit $R_3$ in der Bedeutung eines Essigsäure- oder Methansulfonsäureesters oder eines Wasserstoffatoms,

W eine Hydroxymethylengruppe oder eine

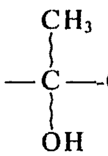
-Gruppe

wobei die OH-Gruppe α- oder β-ständig sein kann,
D eine geradkettige oder verzweigte Alkylengruppe mit 1–5 C-Atomen,
$R_4$ eine Alkylgruppe mit 1–7 C-Atomen

bedeuten, und, falls $R_2$ die Bedeutung eines Wasserstoffatoms hat, deren Salze mit physiologisch verträglichen Basen bedeuten.

Die Verbindungen der Formel I stellen sowohl (5E)- als auch (5Z)-Isomere dar.

Als Alkylgruppen $R_2$ sind solche mit 1–4 C-Atomen, wie z. B. Methyl, Ethyl, Propyl, Isobutyl, Butyl zu nennen.

Als Alkylgruppen $R_4$ kommen gerad- und verzweigtkettige, gesättigte Alkylreste mit 1–7 C-Atomen in Frage. Beispielsweise genannt seien Methyl-, Ethyl-, Propyl-, Butyl-, Isobutyl-, tert.-Butyl-, Pentyl-, Hexyl-, Heptyl-.

Als Alkylengruppe D kommen geradkettige oder verzweigtkettige Alkylenreste mit 1–5 C-Atomen in Frage. Beispielsweise seien genannt: Methylen, Ethylen, 1,2-Propylen, Ethylethylen, Trimethylen, Tetramethylen, Pentamethylen, 1-Methyl-tetramethylen, 1-Methyl-trimethylen.

Zur Salzbildung mit den freien Säuren ($R_2=H$) sind anorganische und organische Basen geeignet, wie sie dem Fachmann zur Bildung Physiologisch verträglicher Salze bekannt sind. Beispielsweise seien genannt: Alkylihydroxide, wie Natrium- und Kaliumhydroxid, Erdalkalihydroxide, wie Calciumhydroxid, Ammoniak, Amine, wie Äthanolamin, Diäthanolamin, Triäthanolamin, N-Methylglucamin, Morpholin, Tris-(hydroxymethyl)-methylamin usw.

Die Erfindung betrifft ferner ein Verfahren zur Herstellung der Carbacyclinderivate der allgemeinen Formel I, dadurch gekennzeichnet, daß man in an sich bekannter Weise in eine Verbindung der allgemeinen Formel II

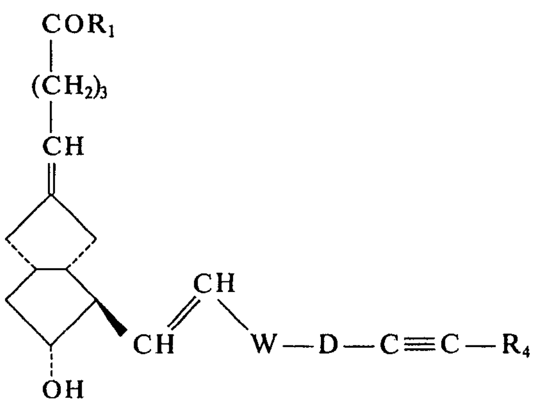
(II)

eine Doppelbindung in 2-Stellung einführt, indem man mit einem Lithiumdialkylamid der allgemeinen Formel III

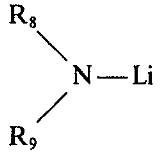
(III)

worin $R_8$ und $R_9$ eine Alkylgruppe mit 1–10 Kohlenstoffatomen oder eine Cycloalkylgruppe mit 3–6 Kohlenstoffatomen oder Trialkylsilylgruppe mit Alkyl in der Bedeutung $C_1$–$C_4$-Alkyl darstellen, umsetzt, mit einer Phenylverbindung der allgemeinen Formel IV

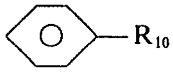
(IV)

worin $R_{10}$ die Reste

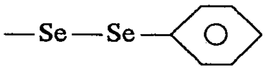 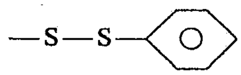 oder —Se—Br

darstellt, behandelt und oxydiert und gegebenenfalls anschließend in beliebiger Reihenfolge Isomere trennt und/oder geschützte Hydroxygruppen freisetzt und/oder freie Hydroxygruppen verestert, veräthert und/oder eine freie Carboxylgruppe verestert und/oder eine veresterte Carboxylgruppe verseift oder eine Carboxylgruppe in eine Gruppe $-CONHR_3$ oder mit einer physiologisch verträglichen Base in ein Salz überführt.

Als Alkylgruppe $R_8$ und $R_9$ kommen alle die für $R_2$ genannten Gruppen in Betracht. Als Cycloalkylgruppen $R_8$ und $R_9$ mit 3–6 C-Atomen sind gemeint: Cyclopropyl, Cyclobutyl, Cyclopentyl, Cyclohexyl.

Die Umsetzung der Verbindungen der allgemeinen Formel II mit einer Lithiumamidverbindung der allgemeinen Formel III, vorzugsweise Lithiumdiisopropylamid, erfolgt in an sich bekannter Weise in einem inerten Lösungsmittel oder Lösungsmittelgemisch wie z. B. Tetrahydrofuran, Diäthyläther, Hexamethylphosphorsäuretriamid, Dimethoxyäthan usw., vorzugsweise Tetrahydrofuran. Die Reaktion wird bei Temperaturen zwischen −100° und 0°C, vorzugsweise −80°C bis −50°C, durchgeführt. Die Umsetzung mit Diphenyldiselenid, Diphenyldisulfid oder Phenylselenylbromid erfolgt bei Temperaturen zwischen −100°C und +30°C, vorzugsweise −70°C und 0°C. Die Oxidation der erhaltenen Selenverbindung erfolgt mit Wasserstoffperoxid in einem inerten Lösungsmittel oder Lösungsmittelgemisch wie z. B. Tetrahydrofuran, Essigsäureäthylester, Essigsäuremethylester, Methanol, Äthanol, Isopropanol usw. bei Temperaturen zwischen −20°C und +60°C, vorzugsweise 0°C bis 30°C, wobei bereits die intermediäre Selenoxidverbindung unter Bildung der $\Delta^2$-Doppelbindung zerfällt.

Die Oxidation der erhaltenen Schwefelverbindung wird zweckmäßigerweise mit Natriumperjodat in Gegenwart eines Alkohols, wie beispielsweise Methanol, Äthanol unter Zusatz von Wasser bei Temperaturen zwischen 0°C und 60°C, vorzugsweise 10°C bis 30°C durchgeführt. Der Zerfall des gebildeten Sulfoxids in die $\Delta^2$-Verbindung der allgemeinen Formel I erfolgt bei Temperaturen zwischen 30°C und 120°C unter Zusatz einer geringen Menge Calciumcarbonat in einem inerten Lösungsmittel wie beispielsweise Toluol, Xylol, Tetrachlorkohlenstoff usw.

Die Verseifung der Carbacyclinester wird nach den dem Fachmann bekannten Methoden durchgeführt, wie beispielsweise mit basischen Katalysatoren.

Die Einführung der Estergruppe $-OR_2$ für $R_1$, bei welcher $R_2$ eine Alkylgruppe mit 1–4 C-Atomen darstellt, erfolgt nach den dem Fachmann bekannten Methoden. Die Carboxyverbindungen werden beispielsweise mit Diazokohlenwasserstoffen in an sich bekannter Weise umgesetzt. Die Veresterung mit Diazokohlenwasserstoffen erfolgt zum Beispiel dadurch, daß man eine Lösung des Diazokohlenwasserstoffes in einem inerten Lösungsmittel, vorzugsweise in Diäthyläther mit einer Carboxyverbindung in dem gleichen oder in einem anderen inerten Lösungsmittel, wie zum Beispiel Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org. Reactions Bd. 8, Seiten 389–394 (1954)].

Die Prostaglandin-Derivate der allgemeinen Formel I mit $R_1$ in der Bedeutung einer Hydroxygruppe können mit geeigneten Mengen der entsprechenden anorganischen Basen unter Neutralisierung in Salze überführt werden. Beispielsweise erhält man beim Lösen der entsprechenden PD-Säuren in Wasser, welches die stöchiometrische Menge der Base enthält, nach Abdampfen des Wassers oder nach Zugabe eines mit Wasser mischbaren Lösungsmittels, zum Beispiel Alkohol oder Aceton, das feste anorganische Salz.

Die Herstellung der Aminsalze erfolgt in üblicher Weise. Dazu wird die PG-Säure zum Beispiel in einem geeigneten Lösungsmittel, wie Äthanol, Aceton, Diäthyläther oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die funktionelle Abwandlung der freien OH-Gruppen erfolgt nach den dem Fachmann bekannten Methoden. Zur Einführung der Ätherschutzgruppen wird beispielsweise mit Dihydropyran in Methylenchlorid oder Chloroform unter Verwendung eines sauren Kondensationsmittels, wie zum Beispiel p-Toluolsulfonsäure, umgesetzt. Das Dihydropyran wird im Überschuß angewandt, vorzugsweise in der 4- bis 10fachen Menge des theoretischen Bedarfs. Die Umsetzung ist normalerweise bei 0°C bis 30°C nach 15 bis 30 Minuten.

Die Freisetzung einer funktionell abgewandelten OH-Gruppe zu den Verbindungen der allgemeinen Formel I erfolgt nach bekannten Methoden. Beispielsweise wird die Abspaltung von Ätherschutzgruppen in einer wäßrigen Lösung einer organischen Säure, wie zum Beispiel Essigsäure, Propionsäure u. a. oder in einer wäßrigen Lösung einer anorganischen Säure, wie zum Beispiel Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind zum Beispiel Alkohole, wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20°C und 80°C durchgeführt.

Die Einführung der Amidgruppe $NHR_3$ für $R_1$ erfolgt nach den dem Fachmann bekannten Methoden. Die Carbonsäuren der allgemeinen Formel I ($R_2$=H) werden zunächst in Gegenwart eines tertiären Amins, wie beispielsweise Triäthylamin, mit Chlorameisensäureisobutylester in das gemischte Anhydrid überführt. Die Umsetzung des gemischten Anhydrids mit dem Alkalisalz des entsprechenden Amids oder mit Ammoniak ($R_3$=H) erfolgt in einem inerten Lösungsmittel oder Lösungsmittelgemisch, wie beispielsweise Tetrahydrofuran, Dimethoxyäthan, Dimethylformamid, Hexamethylphosphorsäuretriamid, bei Temperaturen zwischen −30°C und +60°C, vorzugsweise bei 0°C bis 30°C.

Eine weitere Möglichkeit für die Einführung der Amidgruppe $NHR_3$ für $R_1$ besteht in der Umsetzung einer 1-Carbonsäure der allgemeinen Formel I ($R_2$=H), in der freie Hydroxygruppen gegebenenfalls intermediär geschützt sind, mit Verbindungen der allgemeinen Formel V

$$O{=}C{=}N{-}R_3 \qquad (V)$$

worin $R_3$ die oben angegebene Bedeutung hat.

Die Umsetzung der Verbindung der allgemeinen Formel I ($R_1$=OH) mit einem Isocyanat der allgemeinen Formel V erfolgt gegebenenfalls unter Zusatz eines tertiären Amins, wie z. B. Triäthylamin oder Pyridin. Die Umsetzung kann ohne Lösungsmittel oder in einem inerten Lösungsmittel, vorzugsweise Acetonitril, Tetrahydrofuran, Aceton, Dimethylacetamid, Methylenchlorid, Diäthyläther, Toluol, bei Temperaturen zwischen −80°C bis 100°C, vorzugsweise bei 0°C bis 30°C, vorgenommen werden.

Enthält das Ausgangsprodukt OH-Gruppen im Prostanrest, so werden diese OH-Gruppen auch zur Reaktion gebracht. Werden letztlich Endprodukte gewünscht, die freie Hydroxylgruppen im Prostanrest enthalten, geht man zweckmäßigerweise von Ausgangsprodukten aus, in denen diese durch vorzugsweise leicht abspaltbare Ätherreste intermediär geschützt sind.

Die als Ausgangsmaterial dienenden Verbindungen der allgemeinen Formel II können beispielsweise hergestellt werden, indem man in an sich bekannter Weise ein Keton der Formel VI

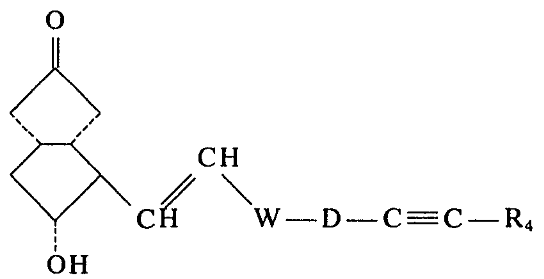

(IV)

worin $R_4$, W und D die oben angegebenen Bedeutungen aufweisen, gegebenenfalls nach Schutz anwesender freier Hydroxygruppen mit einem Wittig-Reagenz der allgemeinen Formel VII

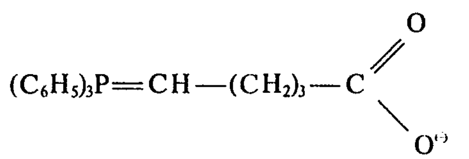

(VII)

umsetzt und gegebenenfalls anschließend die freie Carboxylgruppe verestert. Die Umsetzung der Verbindung der allgemeinen Formel VI mit dem Wittig-Reagenz der allgemeinen Formel VII, das man aus dem entsprechenden Phosphoniumsalz mit Methansulfinylmethylnatrium oder Methansulfinylmethylkalium oder Kalium-tert.-butylat in Dimethylsulfoxid herstellt, wird bei Temperaturen von 0°C bis 100°C, vorzugsweise bei 20°C bis 80°C, in einem aprotischen Lösungsmittel, vorzugsweise Dimethylsulfoxid oder Dimethylformamid, vorgenommen. Die Trennung der dabei erhaltenen Z- und E-konfigurierten Olefine erfolgt auf übliche Art, beispielsweise durch Säulen- oder Schichtchromatographie.

Die Verbindungen dieser Erfindung wirken blutdrucksenkend und bronchodilatatorisch. Sie sind weiterhin geeignet zur Hemmung der Thrombozyten-Aggregation. Folglich stellen die neuen Prostacyclin-Derivate der Formel I wertvolle pharmazeutische Wirkstoffe dar. Darüber hinaus weisen sie bei ähnlichem Wirkungsspektrum, verglichen mit entsprechenden Prostaglandinen, eine höhere Spezifität und vor allem eine wesentlich längere Wirksamkeit auf. Im Vergleich zu $PGI_2$ zeichnen sie sich durch größere Stabilität aus. Die hohe Gewebsspezifität der neuen Carbacycline zeigt sich bei der Untersuchung an glattmuskulären Organen, wie z. B. am Meerschweinchenileum oder an der isolierten Kaninchentrachea, wo eine wesentlich geringere Stimulation zu beobachten ist als bei der Applikation natürlicher Prostaglandine vom E-, A- oder F-Typ.

Die neuen Prostaglandin-Analoga besitzen die für Prostacycline typischen Eigenschaften, wie z. B. Senkung des peripheren arteriellen und koronaren vaskulären Widerstandes, Inhibierung der Thrombozytenaggregation und Auflösung von Plättchenthromben, Senkung des systemischen Blutdruckes ohne zugleich Schlagvolumen und koronare Durchblutung zu senken; Inhibierung der Bronchokonstriktion,

Inhibierung der Magensäuresekretion. Die erfindungsgemäßen Prostacycline können auch in Kombinationen, z. B. mit Beta-Blockern und Diuretika verwendet werden.

Die Dosis der Verbindungen ist 1–1500 µg/kg/Tag, wenn sie am menschlichen Patienten verabreicht werden. Die Einheitsdosis für den pharmazeutischen akzeptablen Träger beträgt 0,01–100 mg.

Bei intravenöser Injektion an wachen, hypertonen Ratten in Dosen von 5, 20 und 100 µg/kg Körpergewicht zeigen die erfindungsgemäßen Verbindungen eine stärkere blutdrucksenkende und länger anhaltende Wirkung als $PGE_2$ und $PGA_2$, ohne wie $PGE_2$ Durchfälle oder $PGA_2$ kardiale Arrhythmien auszulösen.

Bei intravenöser Injektion an narkotisierten Kaninchen zeigen die erfindungsgemäßen Verbindungen in Vergleich zu $PGE_2$ und $PGA_2$ eine stärkere und erheblich länger anhaltende Blutdrucksenkung, ohne daß andere glattmuskuläre Organe oder Organfunktionen beeinflußt werden.

Für die parenterale Verabreichung werden sterile, injizierbare, wäßrige oder ölige Lösungen benutzt. Für die orale Applikation sind beispielsweise Tabletten, Dragees oder Kapseln geeignet.

Die Erfindung betrifft damit auch Arzneimittel auf Basis der Verbindungen der allgemeinen Formel I und üblicher Hilfs- und Trägerstoffe.

Die erfindungsgemäßen Wirkstoffe sollen in Verbindung mit den in der Galenik bekannten und üblichen Hilfsstoffen z. B. zur Herstellung von Blutdrucksenkern dienen.

## Beispiel 1

### (2E,5E)-(16RS)-2,3-Didehydro-16-methyl-18,18,19,19-tetradehydro-6a-carba-prostaglandin-$I_2$-methylester

Zu einer Lösung von 0,215 ml Diisopropylamin in 5 ml Tetrahydrofuran tropft man bei –70°C 1,01 ml einer 1,3molaren Butyllithiumlösung in Hexan und rührt 30 Minuten bei –75°C unter Argon. In diese Lösung tropft man bei –70°C eine Lösung von 0,48 g (5E)-(16RS)-16-Methyl-18,18,19,19-tetradehydro-6a-carba-prostaglandin-$I_2$-methylester-11,15-bis-tetrahydropyran-2-yläther) in 6 ml Tetrahydrofuran, rührt 25 Minuten bei –70°C und tropft anschließend eine Lösung von 420 mg Diphenyldiselenid in 7 ml Tetrahydrofuran zu. Man rührt die Reaktionsmischung 30 Minuten bei –70°C, 30 Minuten bei 0°C, versetzt mit Wasser, säuert mit 3%iger Schwefelsäure auf pH 6 an, extrahiert dreimal mit Äther, wäscht mit Wasser neutral, trocknet über Magnesiumsulfat und dampft im Vakuum ein. Den Rückstand filtriert man mit Äther/Hexan (1+1) über Kieselgel. Dabei erhält man 0,4 g der Selenverbindung als gelbliches Öl.

Zur Oxidation versetzt man eine Lösung von 0,4 g der vorstehend hergestellten Selenverbindung in 5 ml Essigester und 5 ml Tetrahydrofuran mit 0,4 ml 30%iger Wasserstoffperoxidlösung und rührt 4 Stunden bei 25°C. Anschließend verdünnt man mit Essigester, schüttelt einmal mit 5%iger Natriumbicarbonatlösung, wäscht mit Wasser neutral, trocknet über Magnesiumsulfat und dampft im Vakuum ein. Dabei erhält man den Bis-tetrahydropyranäther der Titelverbindung als Öl, den man anschließend 16 Stunden mit 10 ml einer Mischung aus Essigsäure, Wasser, THF (65+35+10) bei 20°C rührt. Man dampft im Vakuum ein und reinigt den Rückstand durch präparative Schichtchromatographie. Mit Äther/Dioxan (9+1) als Laufmittel erhält man 180 mg der Titelverbindung als farbloses Öl.

IR ($CHCl_3$): 3600, 3420 (breit), 2940, 2865, 1720, 1660, 1604, 973 cm.

Das Ausgangsmaterial für die obige Titelverbindung wird wie folgt hergestellt:

## Beispiel 1a

### (5E)-(16RS)-16-Methyl-18,18,19,19-tetradehydro-6a-carba-prostaglandin-$I_2$-11,15-bis-(tetrahydropyran-2-yläther)

Zu einer Lösung von 30,4 g 4-Carboxybutylentriphenylphosphoniumbromid in 60 ml absolutem Dimethylsulfoxid (DMSO) tropft man bei 15°C unter Argon 120 ml einer 1,04molaren Lösung von Methylsulfinylmethylnatrium in DMSO und rührt 30 Minuten bei 20°C. Zur roten Ylenlösung tropft man eine Lösung von 5 g (1R,5S,6R,7R)-7-(Tetrahydropyran-2-yloxy)-6-[(E)-(3S,4RS)-4-methyl-3-(tetrahydropyran-2-yloxy)-oct-1-en-6-inyl]-bicyclo[3.3.0]-octan-3-on in 30 ml absolutem DMSO und rührt 4 Stunden bei 45°C. Das Reaktionsgemisch wird auf Eiswasser gegossen, mit 10%iger Zitronensäurelösung auf pH 4–5 angesäuert und dreimal mit Methylenchlorid extrahiert. Die organische Phase wird mit Sole geschüttelt, über Magnesiumsulfat getrocknet und im Vakuum eingedampft. Nach Chromatographie des Rückstandes an Kieselgel erhält man mit Äther/Hexan (1+1) zunächst 1,2 g (5Z)-(16RS)-16-Methyl-18,18,19,19-tetradehydro-6a-carba-prostaglandin-$I_2$-11,15-bis-(tetrahydropyran-2-yläther) sowie als polare Komponente 2,5 g der Titelverbindung als farbloses Öl.

IR: 3510 (breit), 2960, 2870, 1712, 973/cm.

## Beispiel 1b

(5E)-(16RS)-16-Methyl-18,18,19,19-tetradehydro-6a-carba-prostaglandin-$I_2$-methylester-11,15-bis-(tetrahydropyran-2-yläther)

Eine Lösung von 0,5 g der nach Beispiel 1a hergestellten Säure in 25 ml Methylenchlorid versetzt man unter Rühren bei 0°C tropfenweise mit einer ätherischen Diazomethanlösung bis zur bleibenden Gelbfärbung. Nach Abdampfen des Lösungsmittels reinigt man den Rückstand durch Chromatographie an Kieselgel mit Hexan/Äther (3+2) und erhält 0,48 g der Titelverbindung als Öl.

IR: 1735, 975/cm.

## Beispiel 2

(2E,5E)-(16RS)-2,3-Didehydro-16-methyl-18,18,19,19-tetradehydro-6a-carba-prostaglandin-$I_2$

110 mg des nach Beispiel 1 hergestellten Methylesters rührt man 2,5 Stunden bei 20°C mit 3 ml einer Lösung aus Kaliumhydroxyd in Äthylalkohol und Wasser (Herstellung: Man löst 2 g Kaliumhydroxid in 75 ml Äthanol und 25 ml Wasser). Anschließend säuert man mit 10%iger Zitronensäurelösung auf pH 4 an, extrahiert dreimal mit Methylenchlorid, wäscht den organischen Extrakt einmal mit Sole, trocknet über Magnesiumsulfat und dampft im Vakuum ein. Nach Chromatographie des Rückstandes an Kieselgel mit Methylenchlorid/Isopropanol (9+1) als Elutionsmittel erhält man 65 mg der Titelverbindung als farbloses Öl.

IR: 3600, 3400 (breit), 2930, 2870, 1705, 1655, 1604, 970/cm.

## Beispiel 3

(2E,5Z)-(16RS)-2,3-Didehydro-16-methyl-18,18,19,19-tetradehydro-6a-carba-prostaglandin-$I_2$-methylester

In Analogie zu Beispiel 1 erhält man aus 0,62 g (5Z)-(16RS)-16-Methyl-18,18,19,19-tetradehydro-6a-carba-prostaglandin-$I_2$-methylester-11,15-bis-(tetrahydropyran-2-yläther) (hergestellt aus der entsprechenden Säure bei 0°C mit ätherischer Diazomethanlösung gemäß Beispiel 1b) 0,28 g der Titelverbindung als farbloses Öl.

IR: 3600, 3400 (breit), 2940, 2865, 1720, 1662, 1605, 972/cm.

## Beispiel 4

(2E,5Z)-(16RS)-2,3-Didehydro-16-methyl-18,18,19,19-tetradehydro-6a-carba-prostaglandin-$I_2$

In Analogie zu Beispiel 2 erhält man aus 150 mg des nach Beispiel 3 hergestellten Methylesters 78 mg der Titelverbindung als farbloses Öl.

IR: 3600, 3410 (breit), 2932, 2870, 1704, 1654, 1604, 971/cm.

## Beispiel 5

(2E,5E)-(16RS)-2,3-Didehydro-16,20-dimethyl-18,18,19,19-tetradehydro-6a-carba-prostaglandin-$I_2$-methylester

In Analogie zu Beispiel 1 erhält man aus 1 g (5E)-(16RS)-16,20-Dimethyl-18,18,19,19-tetradehydro-6a-carba-prostaglandin-$I_2$-methylester-11,15-bis-(tetrahydropyran-2-yläther) 390 mg der Titelverbindung als farbloses Öl.

IR: 3610, 3400 (breit), 2940, 2863, 1721, 1660, 1603, 972/cm.

Das Ausgangsmaterial für die obige Titelverbindung wird wie folgt hergestellt:

## Beispiel 5a

(5E)-(16RS)-16,20-Dimethyl-18,18,19,19-tetradehydro-6a-carba-prostaglandin-$I_2$-11,15-bis-(tetrahydropyran-2-yläther)

Zu einer Lösung von 15,2 g 4-Carboxybutyltriphenylphosphoniumbromid in 30 ml Dimethylsulfoxid (DMSO) tropft man bei 15°C unter Argon 60 ml einer 1,04molaren Lösung von Methansulfinylmethylnatrium in DMSO und rührt 30 Minuten bei Raumtemperatur. Zur roten Ylenlösung tropft man eine Lösung von 2,5 g (1R,5S,6R,7R)-7-(Tetrahydropyran-2-yloxy)-6-[(E)-(3S,4RS)-3-(tetrahydropyran-2-yloxy)-4-methyl-non-1-en-6-inyl]-bicyclo[3,3,0]-octan-3-on in 15 ml DMSO und rührt 4 Stunden bei 45°C. Das Reaktionsgemisch wird auf Eiswasser gegossen, mit 10%iger Zitronensäurelösung auf pH 4—5 angesäuert und dreimal mit Methylenchlorid extrahiert. Die organische Phase wird mit Sole geschüttelt, über Magnesiumsulfat getrocknet und im Vakuum eingedampft. Nach Chromatographie des Rückstandes an Kieselgel erhält man mit Äther/Hexan (1+1) zunächst 590 mg (5Z)-(16RS)-16,20-Dimethyl-18,18,19,19-tetradehydro-6a-carba-prostaglandin-$I_2$-11,15-bis-(tetrahydropyran-2-yläther) sowie als polarere Komponente 1,29 g der Titelverbindung als farbloses Öl.
IR: 3500 (breit), 2960, 2870, 1710, 972/cm.

## Beispiel 5b

(5E)-(16RS)-16,20-Dimethyl-18,18,19,19-tetradehydro-6a-carba-prostaglandin-$I_2$-methylester-11,15-bis-(tetrahydropyran-2-yläther)

In Analogie zu Beispiel 1b erhält man aus 0,9 g der nach Beispiel 5a hergestellten Säure 0,85 g der Titelverbindung als Öl.
IR: 2960, 1733, 974/cm.

## Beispiel 6

(2E,5E)-(16RS)-2,3-Didehydro-16,20-dimethyl-18,18,19,19-tetradehydro-6a-carba-prostaglandin-$I_2$

In Analogie zu Beispiel 2 erhält man aus 0,4 g des nach Beispiel 5 hergestellten Methylesters 0,21 g der Titelverbindung als farbloses Öl.
IR: 3605, 3400 (breit), 2930, 2870, 1705, 1653, 1603, 970/cm.

## Beispiel 7

(2E,5Z)-(16RS)-2,3-Didehydro-16,20-dimethyl-18,18,19,19-tetradehydro-6a-carba-prostaglandin-$I_2$-methylester

In Analogie zu Beispiel 1 erhält man aus 0,8 g (5Z)-(16RS)-16,20-Dimethyl-18,18,19,19-tetradehydro-6a-carba-prostaglandin-$I_2$-methylester-11,15-bis-(tetrahydropyran-2-yläther) (hergestellt aus der entsprechenden Säure mit ätherischer Diazomethanlösung gemäß Beispiel 1b) 0,38 g der Titelverbindung als farbloses Öl.
IR: 3600, 3410 (breit), 2942, 2866, 1721, 1662, 1605, 971/cm.

## Beispiel 8

(2E,5Z)-(16RS)-2,3-Didehydro-16,20-dimethyl-18,18,19,19-tetradehydro-6a-carba-prostaglandin-$I_2$

In Analogie zu Beispiel 2 erhält man aus 0,18 g des nach Beispiel 7 hergestellten Methylesters 73 mg der Titelverbindung als farbloses Öl.
IR: 3600, 3400 (breit), 2930, 2870, 1705, 1654, 1603, 970/cm.

## Beispiel 9

(2E,5E)-(16RS)-2,3-Didehydro-16,20-dimethyl-19,19,20,20-tetradehydro-6a-carba-prostaglandin-$I_2$-methylester

In Analogie zu Beispiel 1 erhält man aus 0,6 g (5E)-(16RS)-16,20-Dimethyl-19,19,20,20-tetradehydro-6a-carba-prostaglandin-$I_2$-methylester-11,15-bis(tetrahydropyran-2-yläther) 250 mg der Titelverbindung als farbloses Öl.
IR: 3600, 3400 (breit), 2935, 2860, 1720, 1662, 1605, 972/cm.
Das Ausgangsmaterial für die obige Titelverbindung wird wie folgt hergestellt:

## Beispiel 9a

(5E)-(16RS)-16,20-Dimethyl-19,19,20,20-tetradehydro-6a-carba-prostaglandin-$I_2$-11,15-bis-(tetrahydropyran-2-yläther)

In Analogie zu Beispiel 1a erhält man aus 3 g (1R,5S,6R,7R)-7-(Tetrahydropyran-2-yloxy)-6-[(E)-(3S,4RS)-3-(tetrahydropyran-2-yloxy)-4-methyl-non-1-en-6-inyl]-bicyclo[3.3.0]octan-3-on 650 mg (5Z)-(16RS)-16,20-Dimethyl-19,19,20,20-tetradehydro-6a-carba-prostaglandin-$I_2$-11,15-bis-(tetrahydropyran-2-yläther) und als polarere Komponente 1,6 g der Titelverbindung als farbloses Öl.
IR: 3510 (breit), 2960, 2870, 1708, 971/cm.

## Beispiel 9b

(5E)-(16RS)-16,20-Dimethyl-19,19,20,20-tetradehydro-6a-carba-prostaglandin-$I_2$-methylester-11,15-bis-(tetrahydropyran-2-yläther)

In Analogie zu Beispiel 1b erhält man aus 1,6 g der nach Beispiel 9a hergestellten Säure 1,45 g des Methylesters als farbloses Öl.
IR: 2960, 1734, 973/cm.

## Beispiel 10

(2E,5E)-(16RS)-2,3-Didehydro-16,20-dimethyl-19,19,20,20-tetradehydro-6a-carba-prostaglandin-$I_2$

In Analogie zu Beispiel 2 erhält man aus 0,2 g des nach Beispiel 9 hergestellten Methylesters 0,12 g der Titelverbindung als farbloses Öl.
IR: 3600, 3400 (breit), 2932, 2868, 1704, 1652, 1602, 972/cm.

## Beispiel 11

(2E,5Z)-(16RS)-2,3-Didehydro-16,20-dimethyl-19,19,20,20-tetradehydro-6a-carba-prostaglandin-$I_2$-methylester

In Analogie zu Beispiel 1 erhält man aus 0,95 g (5Z)-(16RS)-16,20-Dimethyl-19,19,20,20-tetradehydro-6a-carba-prostaglandin-$I_2$-methylester-11,15-bis-(tetrahydropyran-2-yläther) (hergestellt aus der entsprechenden Säure mit ätherischer Diazomethanlösung gemäß Beispiel 1b) 0,4 g der Titelverbindung als farbloses Öl.
IR: 3600, 3400 (breit), 2940, 2865, 1721, 1663, 1605, 971/cm.

## Beispiel 12

(2E,5Z)-(16RS)-2,3-Didehydro-16,20-dimethyl-19,19,20,20-tetradehydro-6a-prostaglandin-$I_2$

In Analogie zu Beispiel 2 erhält man aus 0,3 g des nach Beispiel 11 hergestellten Methylesters 0,18 g der Titelverbindung als farbloses Öl.
IR: 3600, 3410 (breit), 2930, 2871, 1704, 1653, 1603, 971/cm.

## Beispiel 13

(2E,5E)-(15RS)-2,3-Didehydro-15-methyl-18,18,19,19-tetradehydro-6a-carba-prostaglandin-$I_2$-methylester

In Analogie zu Beispiel 1 erhält man aus 0,5 g (5E)-(15RS)-15-Methyl-18,18,19,19-tetradehydro-6a-carba-prostaglandin-$I_2$-methylester-11,15-bis-(tetrahydropyran-2-yl)-äther 245 mg der Titelverbindung als farbloses Öl.
IR: 3600, 3405, 2954, 2858, 1720, 1660, 1600, 978/cm.
Das Ausgangsmaterial für die obige Titelverbindung wird wie folgt hergestellt:

Beispiel 13a

(5E)-(15RS)-15-Methyl-18,18,19,19-tetradehydro-6a-carba-prostaglandin-$I_2$-11,15-bis-(tetrahydropyran-2-yl)-äther

In Analogie zu Beispiel 1a erhält man aus 2,5 g (1R,5S,6R,7R)-7-(Tetrahydropyran-2-yloxy)-6-[(E)-(3RS)-3-(tetrahydropyran-2-yloxy)-oct-1-en-6-inyl]-bicyclo[3.3.0]octan-3-on 430 mg (5Z)-(15RS)-15-Methyl-18,18,19,19-tetradehydro-6a-carba-prostaglandin-$I_2$-11,15-bis-(tetrahydropyran-2-yl)-äther und als polarere Komponente 1,2 g der Titelverbindung als farbloses Öl.
IR: 3580, 2965, 1710, 978/cm.

Beispiel 13b

(5E)-(15RS)-15-Methyl-18,18,19,19-tetradehydro-6a-carba-prostaglandin-$I_2$-methylester-11,15-bis-(tetrahydropyran-2-yl)-äther

In Analogie zu Beispiel 1b erhält man aus 1,2 g der nach Beispiel 13a hergestellten Säure 1,05 g des Methylesters als farbloses Öl.
IR: 2958, 2862, 1735, 976/cm.

Beispiel 14

(2E,5E)-(15RS)-2,3-Didehydro-15-methyl-18,18,19,19-tetradehydro-6a-carba-prostaglandin-$I_2$

In Analogie zu Beispiel 2 erhält man aus 120 mg des nach Beispiel 13 hergestellten Methylesters 80 mg der Titelverbindung als farbloses Öl.
IR: 3605, 3400 (breit), 2948, 2862, 1700, 1650, 1602, 976/cm.

Beispiel 15

(2E,5E)-2,3-Didehydro-18,18,19,19-tetradehydro-6a-carba-prostaglandin-$I_2$-n-butylester

In Analogie zu Beispiel 1 erhält man aus 800 mg (5E)-18,18,19,19-Tetradehydro-6a-carba-prostaglandin-$I_2$-n-butylester-11,15-bis-(tetrahydropyran-2-yl)-äther 370 mg der Titelverbindung als farbloses Öl.
IR: 3600, 3410, 2960, 2852, 1722, 1662, 1602, 976/cm.
Das Ausgangsmaterial für die obige Titelverbindung wird wie folgt hergestellt:

Beispiel 15a

(5E)-18,18,19,19-Tetradehydro-6a-carba-prostaglandin-$I_2$-11,15-bis-(tetrahydropyran-2-yl)-äther

In Analogie zu Beispiel 1a erhält man aus 2,45 g (1R,5S,6R,7R)-7-(Tetrahydropyran-2-yloxy)-6-[(E)-(3S)-3-(tetrahydropyran-2-yloxy)-oct-1-en-6-inyl]-bicyclo[3.3.0]octan-3-on 400 mg (5Z)-18,18,19,19-Tetradehydro-6a-carba-prostaglandin-$I_2$-11,15-bis-(tetrahydropyran-2-yl)-äther und als polarere Komponente 1,21 g der Titelverbindung als farbloses Öl.
IR: 3585, 3400, 2960, 2854, 1710, 976/cm.

Beispiel 15b

(5E)-18,18,19,19-Tetradehydro-6a-carba-prostaglandin-$I_2$-n-butylester-11,15-bis-(tetrahydropyran-2-yl)-äther

Zu einer Lösung von 1 g der nach Beispiel 15a hergestellten Säure in 50 ml Äther tropft man bei 0°C eine 1M ätherische Lösung von Diazobutan und verfolgt die Reaktion dünnschichtchromatographisch (Kieselgelplatten mit Essigester/Hexan [3+7] als Laufmittel). Nach vollständiger Umsetzung dampft man im Vakuum ein und reinigt den Rückstand durch Säulenchromatographie an Kieselgel, wobei Hexan mit steigendem Essigestergradienten verwendet wird. Man erhält 0,91 g der Titelverbindung als hellgelbes Öl.
IR: 2962, 2860, 1740, 978/cm.

Beispiel 16

(2E,5E)-2,3-Didehydro-18,18,19,19-tetradehydro-6a-carba-prostaglandin-$I_2$

In Analogie zu Beispiel 2 erhält man aus 250 mg des nach Beispiel 15 hergestellten Butylesters 100 mg der Titelverbindung als Öl.
IR: 3605, 3410, 2952, 2860, 1702, 1652, 1602, 976/cm.

Beispiel 17

(2E,5E)-(16RS)-2,3-Didehydro-16-methyl-18,18,19,19-tetradehydro-6a-carba-prostaglandin-$I_2$-N-methansulfonyl-carboxamid

Eine Lösung von 358 mg (2E,5E)-(16RS)-2,3-Didehydro-16-methyl-18,18,19,19-tetradehydro-6a-carba-prostaglandin-$I_2$ in 8 ml Dimethylformamid wird bei 0°C mit 160 mg Chlorameisensäureisobutylester und 120 mg Triäthylamin versetzt. Nach 30 Minuten werden 480 mg des Natriumsalzes von Methansulfonamid (hergestellt aus Methansulfonamid und Natriummethylat) und 2 ml Hexamethylphosphorsäuretriamid zugesetzt und 3 Stunden bei 25°C gerührt. Anschließend gibt man das Reaktionsgemisch auf Citratpuffer (pH 5), extrahiert mehrere Male mit Essigester, wäscht die organische Phase mit Sole, trocknet über Magnesiumsulfat und dampft im Vakuum ein. Nach Chromatographie des Rückstandes an Kieselgel mit Methylenchlorid/Isopropanol (9+1) erhält man 210 mg der Titelverbindung als Öl.
IR: 3600, 3450, 1705, 1645, 976/cm.

Beispiel 18

(2E,5E)-(16RS)-2,3-Didehydro-16-methyl-18,18,19,19-tetradehydro-6a-carba-prostaglandin-$I_2$-N-acetyl-amid

Zu einer Lösung von 248 mg (2E,5E)-(16RS)-2,3-Didehydro-16-methyl-18,18,19,19-tetradehydro-6a-carba-prostaglandin-$I_2$-11,15-bis-(tetrahydropyran-2-yl)-äther in 8 ml Acetonitril gibt man bei 25°C 65 mg Triäthylamin, kühlt dann auf 0°C und tropft eine Lösung von 42 mg Acetylisocyanat in 4 ml Acetonitril zu. Man rührt dann 2 Stunden bei 5°C, engt im Vakuum ein, verdünnt mit 50 ml Wasser, säuert durch Zugabe von 1N Schwefelsäure auf pH 4 an, extrahiert mit Äther, wäscht die organische Phase mit Sole, trocknet über Magnesiumsulfat und dampft im Vakuum ein. Zur Abspaltung der Schutzgruppen rührt man den Rückstand mit 7 ml Eisessig/Wasser/Tetrahydrofuran (65/35/10) über Nacht bei 30°C und dampft im Vakuum ein. Der Rückstand wird an Kieselgel mit Methylenchlorid/5% Isopropanol chromatographiert. Man erhält 150 mg der Titelverbindung als Öl.
IR: 3600, 3440, 1698, 1645, 976/cm.

Beispiel 19

(2E,5E)-(16RS)-2,3-Didehydro-16-methyl-18,18,19,19-tetradehydro-6a-carba-prostaglandin-$I_2$-carboxamid

Man löst 398 mg (2E,5E)-(16RS)-2,3-Didehydro-16-methyl-18,18,19,19-tetradehydro-6a-carba-prostaglandin-$I_2$ in 5 ml Tetrahydrofuran und fügt 160 mg Triäthylamin und 180 mg Chlorameisensäureisobutylester zu und läßt eine Stunde bei 0°C stehen. Sodann wird für 10 Minuten bei 0°C Ammoniakgas eingeleitet und anschließend eine Stunde bei 25°C belassen. Zur Aufarbeitung wird mit 100 ml Wasser verdünnt, mehrmals mit Methylenchlorid extrahiert, die vereinigten Extrakte mit Sole geschüttelt, über Magnesiumsulfat getrocknet und im Vakuum eingedampft. Man reinigt durch Chromatographie an Kieselgel mit Chloroform/Essigester (8+2) und erhält 300 mg der Titelverbindung als Öl.
IR: 3600, 3510, 3410, 2958, 2862, 1655, 976/cm.

Beispiel 20

(2E,5E)-(16RS)-2,3-Didehydro-16-methyl-18,18,19,19-tetradehydro-6a-carba-prostaglandin-$I_2$-tris(hydroxymethyl)aminomethansalz

Zu einer Lösung von 100 mg (2E,5E)-(16RS)-2,3-Didehydro-16-methyl-18,18,19,19-tetradehydro-6a-carba-prostaglandin-$I_2$ in 18 ml Acetonitril gibt man bei 65°C eine Lösung von 30 mg Tris-(hydroxymethyl)-aminomethan in 0,1 ml Wasser. Man läßt unter Rühren abkühlen, dekantiert nach 16 Stunden vom Lösungsmittel und trocknet den Rückstand im Vakuum. Man erhält 80 mg der Titelverbindung als zähes Öl.

**Patentansprüche für die Vertragsstaaten BE, CH, DE, FR, GB, IT, LI, LU, NL, SE**

1. Carbacyclinderivate der allgemeinen Formel I

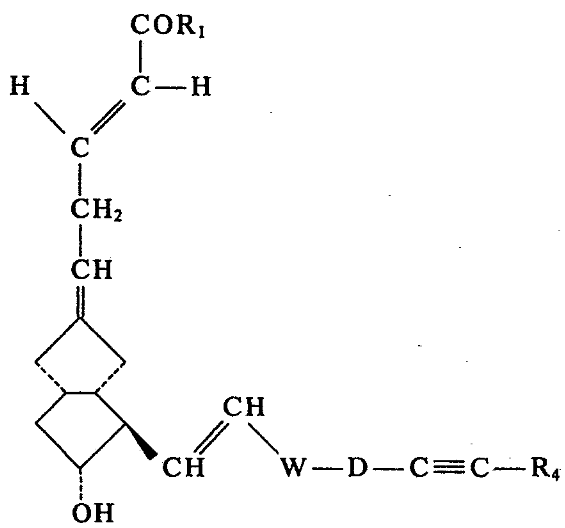

(I)

worin

$R_1$ den Rest $OR_2$, wobei $R_2$ Wasserstoff oder Alkyl mit 1–4 C-Atomen bedeuten kann, oder den Rest $NHR_3$ mit $R_3$ in der Bedeutung eines Essigsäure- oder Methansulfonsäurerestes oder eines Wasserstoffatoms,

W eine Hydroxymethylengruppe oder eine

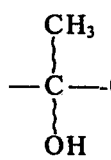

-Gruppe

wobei die OH-Gruppe $\alpha$- oder $\beta$-ständig sein kann,

D eine geradkettige oder verzweigte Alkylengruppe mit 1–5 C-Atomen,

$R_4$ eine Alkylgruppe mit 1–7 C-Atomen

bedeuten, und, falls $R_2$ die Bedeutung eines Wasserstoffatoms hat, deren Salze mit physiologisch verträglichen Basen.

2. Verfahren zur Herstellung der Carbacyclinderivate der allgemeinen Formel I, dadurch gekennzeichnet, daß man in an sich bekannter Weise in eine Verbindung der allgemeinen Formel II

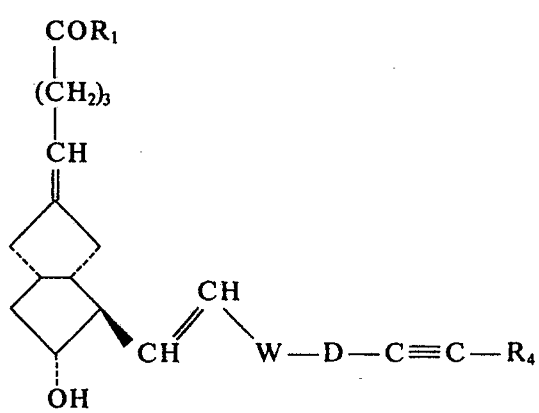

(II)

eine Doppelbindung in 2-Stellung einführt, indem man mit einem Lithiumdialkylamid der allgemeinen Formel III

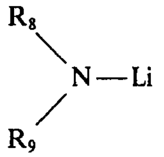

(III)

worin $R_8$ und $R_9$ eine Alkylgruppe mit 1—10 Kohlenstoffatomen oder eine Cycloalkylgruppe mit 3–6 Kohlenstoffatomen oder Trialkylsilylgruppe mit Alkyl in der Bedeutung $C_1$—$C_2$4-Alkyl darstellen, umsetzt, mit einer Phenylverbindung der allgemeinen Formel IV

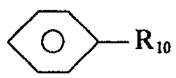

(IV)

worin $R_{10}$ die Reste

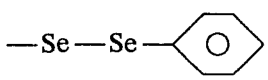 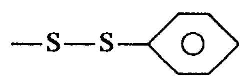 oder —Se—Br

darstellt, behandelt und oxydiert und gegebenenfalls anschließend in beliebiger Reihenfolge Isomere trennt und/oder geschützte Hydroxygruppen freisetzt und/oder freie Hydroxygruppen verestert, veräthert und/oder eine freie Carboxylgruppe verestert und/oder eine veresterte Carboxylgruppe verseift oder eine Carboxylgruppe in eine Gruppe —$CONHR_3$ oder mit einer physiologisch verträglichen Base in ein Salz überführt.

3. Arzneimittel, bestehend aus einer oder mehreren Verbindungen gemäß Anspruch 1 und üblichen Hilfs- und Trägerstoffen.

4. (2E,5E)-(16RS)-2,3-Didehydro-16-methyl-18,18,19,19-tetradehydro-6a-carba-prostaglandin-$I_2$.

5. (2E,5Z)-(16RS)-2,3-Didehydro-16-methyl-18,18,19,19-tetradehydro-6a-carba-prostaglandin-$I_2$.

6. (2E,5E)-(16RS)-2,3-Didehydro-16,20-dimethyl-18,18,19,19-tetradehydro-6a-carba-prostaglandin-$I_2$.

7. (2E,5Z)-(16RS)-2,3-Didehydro-16,20-dimethyl-18,18,19,19-tetradehydro-6a-carba-prostaglandin-$I_2$.

8. (2E,5E)-(16RS)-2,3-Didehydro-16,20-dimethyl-19,19,20,20-tetradehydro-6a-carba-prostaglandin-$I_2$.

9. (2E,5Z)-(16RS)-2,3-Didehydro-16,20-dimethyl-19,19,20,20-tetradehydro-6a-carba-prostaglandin-$I_2$.

10. (2E,5E)-(15RS)-2,3-Didehydro-15-methyl-18,18,19,19-tetradehydro-6a-carba-prostaglandin-$I_2$.

11. (2E,5E)-2,3-Didehydro-18,18,19,19-tetradehydro-6a-carba-prostaglandin-$I_2$-n-butylester.

12. (2E,5E)-2,3-Didehydro-18,18,19,19-tetradehydro-6a-carba-prostaglandin-$I_2$.

13. (2E,5E)-(16RS)-2,3-Didehydro-16-methyl-18,18,19,19-tetradehydro-6a-carba-prostaglandin-$I_2$-N-methansulfonyl-carboxamid.

14. (2E,5E)-(16RS)-2,3-Didehydro-16-methyl-18,18,19,19-tetradehydro-6a-carba-prostaglandin-$I_2$-N-acetyl-amid.

15. (2E,5E)-(16RS)-2,3-Didehydro-16-methyl-18,18,19,19-tetradehydro-6a-carba-prostaglandin-$I_2$-carboxamid.

16. (2E,5E)-(16RS)-2,3-Didehydro-16-methyl-18,18,19,19-tetradehydro-6a-carba-prostaglandin-$I_2$-tris(hydroxymethyl)-aminomethansalz.

**Patentanspruch für den Vertragsstaat AT**

Verfahren zur Herstellung von Carbacyclinderivaten der allgemeinen Formel I

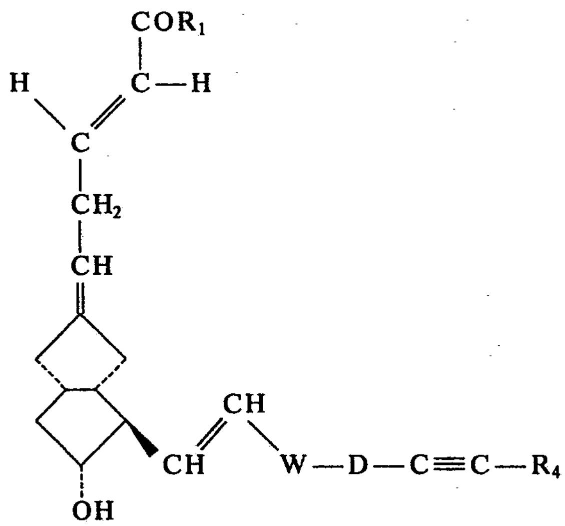

(I)

worin

$R_1$ den Rest $OR_2$, wobei $R_2$ Wasserstoff oder Alkyl mit 1–4 C-Atomen bedeuten kann oder den Rest $NHR_3$ mit $R_3$ in der Bedeutung eines Essigsäure- oder Methansulfonsäurerestes oder eines Wasserstoffatoms,

W eine Hydroxymethylengruppe oder eine

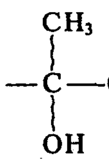
Gruppe

wobei die OH-Gruppe α- oder β-ständig sein kann,

D eine geradkettige oder verzweigte Alkylengruppe mit 1–5 C-Atomen,

$R_4$ eine Alkylgruppe mit 1–7 C-Atomen

bedeuten, und, falls $R_2$ die Bedeutung eines Wasserstoffatoms hat, deren Salze mit physiologisch verträglichen Basen,
dadurch gekennzeichnet, daß man in an sich bekannter Weise in eine Verbindung der allgemeinen Formel II

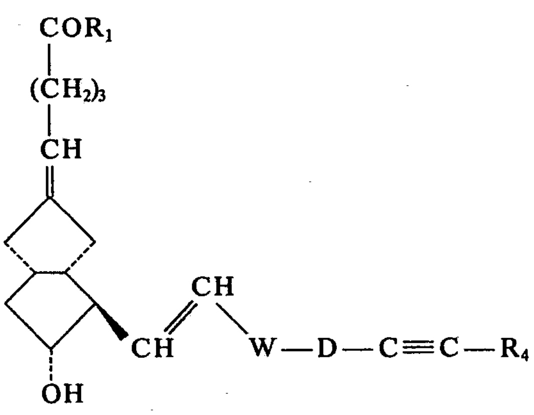

(II)

eine Doppelbindung in 2-Stellung einführt, indem man mit einem Lithiumdialkylamid der allgemeinen

Formel III

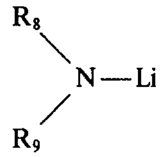

(III)

worin $R_8$ und $R_9$ eine Alkylgruppe mit 1–10 Kohlenstoffatomen oder eine Cycloalkylgruppe mit 3–6 Kohlenstoffatomen oder Trialkylsilylgruppe mit Alkyl in der Bedeutung $C_1$–$C_2$4-Alkyl darstellen, umsetzt, mit einer Phenylverbindung der allgemeinen Formel IV

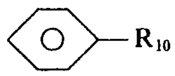

(IV)

worin $R_{10}$ die Reste

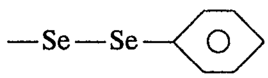

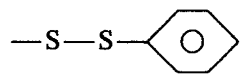

oder —Se—Br

darstellt, behandelt und oxydiert und gegebenenfalls anschließend in beliebiger Reihenfolge Isomere trennt und/oder geschützte Hydroxygruppen freisetzt und/oder freie Hydroxygruppen verestert, veräthert und/oder eine freie Carboxylgruppe verestert und/oder eine veresterte Carboxylgruppe verseift oder eine Carboxylgruppe in eine Gruppe $-CONHR_3$ oder mit einer physiologisch verträglichen Base in ein Salz überführt.

**Claims for the contracting states: BE, CH, DE, FR, GB, IT, LI, LU, NL, SE**

1. Carbacyclin derivatives of the general formula I

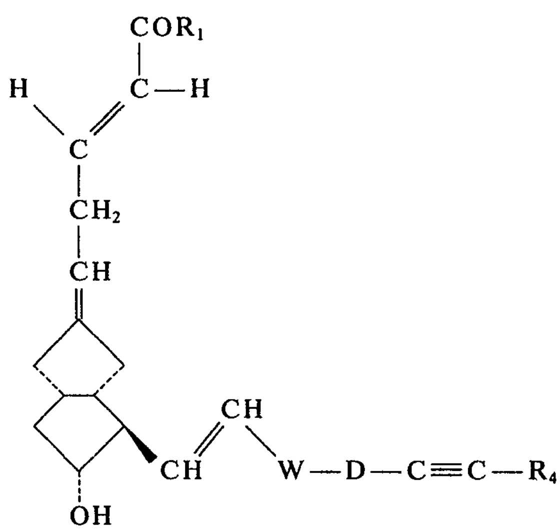

(I)

wherein

$R_1$ is the group $OR_2$, where $R_2$ is hydrogen or alkyl of 1–4 carbon atoms, or the group $NHR_3$, where $R_3$ is acetyl, methanesulfonyl or hydrogen,

W is a hydroxymethylene group or a

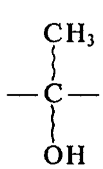

group, in which the OH is in the $\alpha$- or $\beta$-configuration,

D is a straight or branched chain alkylene group of 1–5 carbon atoms,

$R_4$ is alkyl of 1–7 carbon atoms,

and when $R_2$ is hydrogen, salts of these compounds with physiologically acceptable bases.

2. Process for the preparation of the carbacyclin derivatives of general formula I, characeterised in that a double bond is introduced in known manner in the 2-position into a compound of general formula II

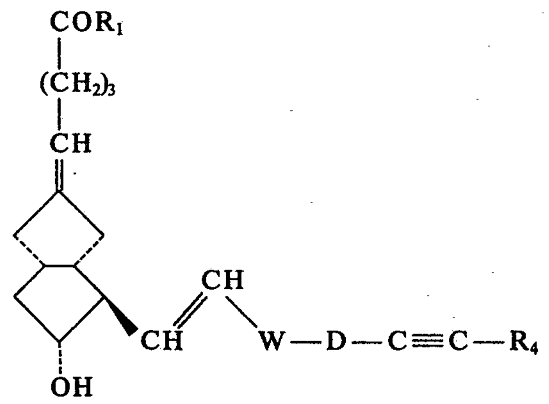

(II)

by reacting it with a lithium alkylamine of general formula III

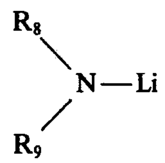

(III)

wherein $R_8$ and $R_9$ are alkyl of 1–10 carbon atoms, cycloalkyl of 3–6 carbon atoms or a trialkylsilyl group in which the alkyl is of 1–4 carbon atoms, treating it with a phenyl compound of general formula IV

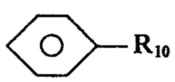

(IV)

in which $R_{10}$ is the group

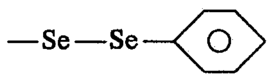 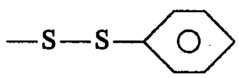 or $-Se-Br$

and oxidising it, and then optionally, and in an arbitrary sequence separating the isomers, and/or freeing protected hydroxy groups, and/or esterifying or etherifying free hydroxy groups, and/or esterifying a free carboxyl group and/or saponifying an esterified carboxyl group or converting a carboxyl group into a $-CONHR_3$ group or into a salt with a physiologically acceptable base.

3. A pharmaceutical composition comprising one or more compounds according to claim 1 and conventional diluents or carriers.

4. (2E,5E)-(16RS)-2,3-Didehydro-16-methyl-18,18,19,19-tetradehydro-6a-carba-prostaglandin-$I_2$.

5. (2E,5Z)-(16RS)-2,3-Didehydro-16-methyl-18,18,19,19-tetradehydro-6a-carba-prostaglandin-$I_2$.

6. (2E,5E)-(16RS)-2,3-Didehydro-16,20-dimethyl-18,18,19,19-tetradehydro-6a-carba-prostaglandin-$I_2$.

7. (2E,5Z)-(16RS)-2,3-Didehydro-16,20-dimethyl-18,18,19,19-tetradehydro-6a-carba-prostaglandin-$I_2$.

8. (2E,5E)-(16RS)-2,3-Didehydro-16,20-dimethyl-19,19,20,20-tetradehydro-6a-carba-prostaglandin-$I_2$.

9. (2E,5Z)-(16RS)-2,3-Didehydro-16,20-dimethyl-19,19,20,20-tetradehydro-6a-carba-prostaglandin-$I_2$.

10. (2E,5E)-(15RS)-2,3-Didehydro-15-methyl-18,18,19,19-tetradehydro-6a-carba-prostaglandin-$I_2$.

11. (2E,5E)-2,3-Didehydro-18,18,19,19-tetradehydro-6a-carba-prostaglandin-$I_2$ n-butyl ester.

12. (2E,5E)-2,3-Didehydro-18,18,19,19-tetradehydro-6a-carba-prostaglandin-$I_2$.

13. (2E,5E)-(16RS)-2,3-Didehydro-16-methyl-18,18,19,19-tetradehydro-6a-carba-prostaglandin-$I_2$ N-methanesulfonylcarboxamide.

14. (2E,5E)-(16RS)-2,3-Didehydro-16-methyl-18,18,19,19-tetradehydro-6a-carba-prostaglandin-$I_2$ N-acetylamide.
15. (2E,5E)-(16RS)-2,3-Didehydro-16-methyl-18,18,19,19-tetradehydro-6a-carba-prostaglandin-$I_2$-carboxamide.
16. (2E,5E)-(16RS)-2,3-Didehydro-16-methyl-18,18,19,19-tetradehydro-6a-carba-prostaglandin-$I_2$ tris(hydroxymethyl)aminomethane salt.

**Claim for the contracting state: Austria**

1. Process for the preparation of the carbacyclin derivatives of the general formula I

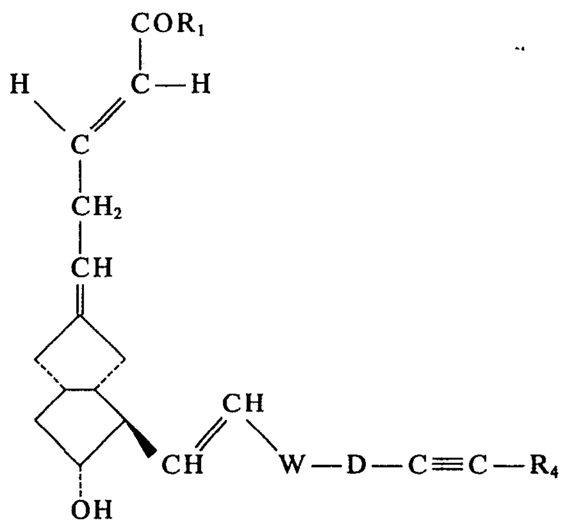

(I)

wherein

$R_1$ is the group $OR_2$, where $R_2$ is hydrogen or alkyl of 1–4 carbon atoms, or the group $NHR_3$, where $R_3$ is acetyl, methanesulfonyl or hydrogen,
W is a hydroxymethylene group or a

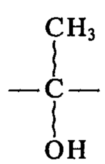

group, in which the OH is in the $\alpha$- or $\beta$-configuration,
D is a straight or branched chain alkylene group of 1–5 carbon atoms,
$R_4$ is alkyl of 1–7 carbon atoms,

and when $R_2$ is hydrogen, salts of these compounds with physiologically acceptable bases, characterised in that a double bond is introduced in known manner in the 2-position into a compound of general formula II

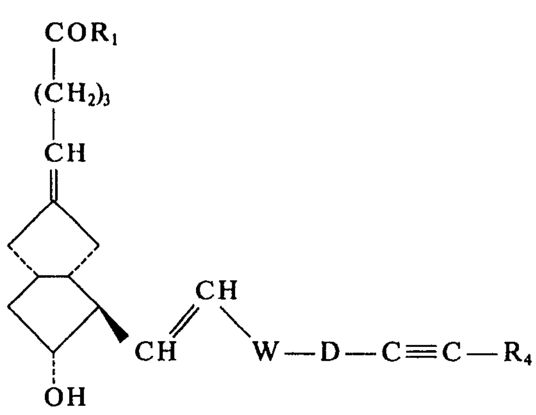

(II)

by reacting it with a lithium alkylamine of general formula III

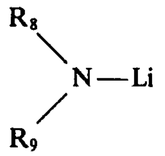

(III)

wherein $R_8$ and $R_9$ are alkyl of 1–10 carbon atoms, cycloalkyl of 3–6 carbon atoms or a trialkylsilyl group in which the alkyl is of 1–4 carbon atoms, treating it with a phenyl compound of general formula IV

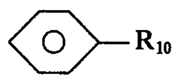

(IV)

in which $R_{10}$ is the group

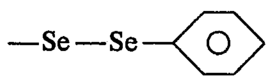 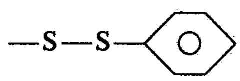 or —Se—Br

and oxidising it, and then optionally, and in an arbitrary sequence separating the isomers, and/or freeing protected hydroxy groups, and/or esterifying or etherifying free hydroxy groups, and/or esterifying a free carboxyl group and/or saponifying an esterified carboxyl group or converting a carboxyl group into a $-CONHR_3$ group or into a salt with a physiologically acceptable base.

**Revendications pour les Etats contractants: BE, CH, DE, FR, GB, IT, LI, LU, NL, SE**

1. Dérivés de la carbacycline répondant à la formule générale I:

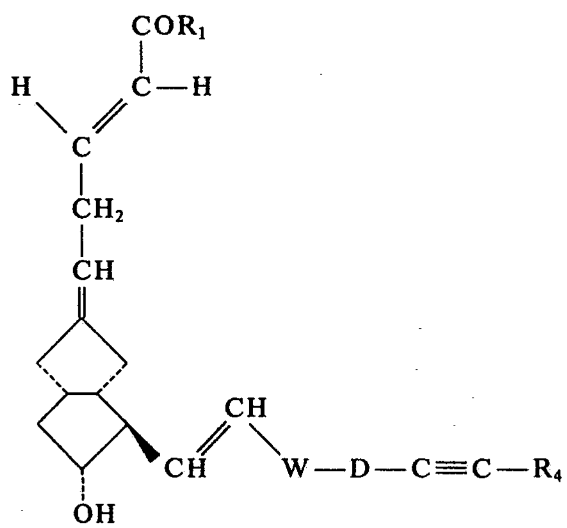

(I)

dans laquelle

$R_1$ représente soit un radical $-OR_2$ dans lequel $R_2$ désigne l'hydrogène ou un alkyle contenant de 1 à 4 atomes de carbone, soit un radical $-NHR_3$ dans lequel $R_3$ désigne le radical de l'acide acétique ou celui de l'acide méthane-sulfonique ou encore un atome d'hydrogène,

W représente un radical hydroxy-méthylène ou un radical

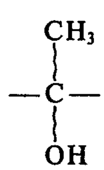

dont le radical –OH peut avoir la configuration $\alpha$ ou la configuration $\beta$,

D représente un radical alkylène, linéaire ou ramifié, contenant de 1 à 5 atomes de carbone, et

$R_4$ représente un radical alkyle contenant de 1 à 7 atomes de carbone,

et également, dans le cas où $R_2$ représente un atome d'hydrogène, les sels que forment ces composés avec des bases acceptables du point de vue physiologique.

2. Procédé de préparation des dérivés de la carbacycline de formule générale I, procédé caractérisé en ce qu'on introduit, de manière connue, une double liaison en position 2 dans un composé de formule générale II:

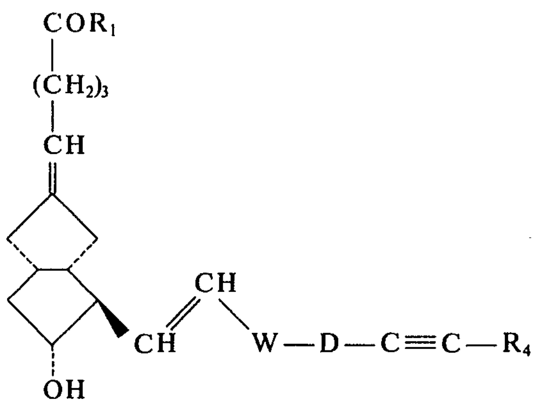

(II)

cela en faisant réagir un dialkylamidure de lithium répondant à la formule générale III:

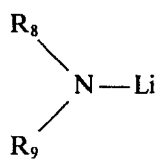

(III)

dans laquelle $R_8$ et $R_9$ représentent chacun un alkyle contenant de 1 à 10 atomes de carbone, un cycloalkyle contenant de 3 à 6 atomes de carbone ou un trialkylsilyle dont chacun des alkyles contient de 1 à 4 atomes de carbone, en traitant par un composé phénylique répondant à la formule générale IV:

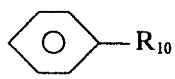

(IV)

dans laquelle $R_{10}$ représente un radical

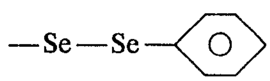 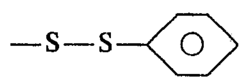 ou —Se—Br

et en oxydant, et ensuite, le cas échéant, on effectue dans l'ordre que l'on veut les opérations suivantes: on sépare des isomères et/ou on libère des radicaux hydroxy protégés et/ou estérifie ou éthérifie des radicaux hydroxy libres et/ou on estérifie un radical carboxy libre et/ou on saponifie un radical carboxy estérifié ou on transforme un radical carboxy en un radical $-CONHR_3$ ou, au moyen d'une base acceptable du point de vue physiologique, en un sel.

3. Médicament renfermant un ou plusieurs composés selon la revendication 1 ainsi que des adjuvants et des excipients usuels.

4. Didéhydro-2,3 méthyl-16 tétradéhydro-18,18,19,19 carba-6a prostaglandine $I_2$ (2E,5E)-(16RS).

5. Didéhydro-2,3 méthyl-16 tétradéhydro-18,18,19,19 carba-6a prostaglandine $I_2$ (2E,5Z)-(16RS).

6. Didéhydro-2,3 diméthyl-16,20 tétradéhydro-18,18,19,19 carba-6a prostaglandine $I_2$ (2E,5E)-(16RS).

7. Didéhydro-2,3 diméthyl-16,20 tétradéhydro-18,18,19,19 carba-6a prostaglandine $I_2$ (2E,5Z)-(16RS).

8. Didéhydro-2,3 diméthyl-16,20 tétradéhydro-19,19,20,20 carba-6a prostaglandine $I_2$ (2E,5E)-(16RS).

9. Didéhydro-2,3 diméthyl-16,20 tétradéhydro-19,19,20,20 carba-6a prostaglandine $I_2$ (2E,5Z)-(16RS).

10. Didéhydro-2,3 méthyl-15 tétradéhydro-18,18,19,19 carba-6a prostaglandine $I_2$ (2E,5E)-(15RS).

11. Ester n-butylique de la didéhydro-2,3 tétradéhydro-18,18,19,19 carba-6a prostaglandine $I_2$ (2E,5E).

12. Didéhydro-2,3 tétradéhydro-18,18,19,19 carba-6a prostaglandine $I_2$ (2E,5E).

13. N-Méthane-sulfonyl-amide de la didéhydro-2,3 méthyl-16 tétradéhydro-18,18,19,19 carba-6a prostaglandine $I_2$ (2E,5E)-(16RS).

14. N-Acétyl-amide de la didéhydro-2,3 méthyl-16 tétradéhydro-18,18,19,19 carba-6a prostaglandine $I_2$ (2E,5E)-(16RS).

15. Amide de la didéhydro-2,3 méthyl-16 tétradéhydro-18,18,19,19 carba-6a prostaglandine $I_2$ (2E,5E)-(16RS).

16. Sel de la didéhydro-2,3 méthyl-16 tétradéhydro-18,18,19,19 carba-6a prostaglandine $I_2$ (2E,5E)-(16RS) avec le tris-(hydroxyméthyl)-amino-méthane.

**Revendication pour l'Etat contractant: AT**

Procédé de préparation des dérivés de la carbacycline de formule générale I,

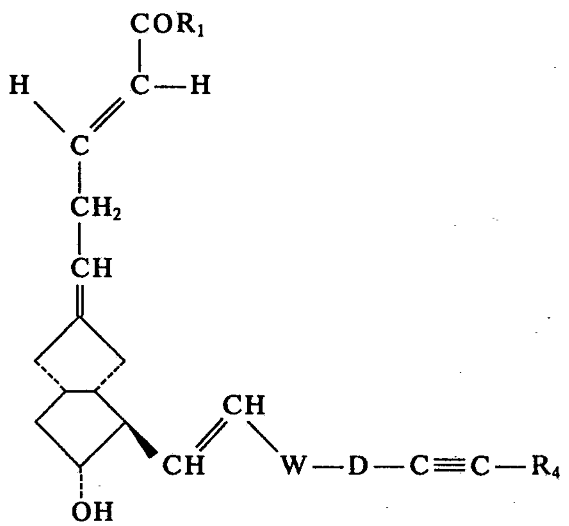

(I)

dans laquelle

$R_1$ représente soit un radical $-OR_2$ dans lequel $R_2$ désigne l'hydrogène ou un alkyle contenant de 1 à 4 atomes de carbone, soit un radical $-NHR_3$ dans lequel $R_3$ désigne le radical de l'acide acétique ou celui de l'acide méthane-sulfonique ou encore un atome d'hydrogène,

W représente un radical hydroxy-méthylène ou un radical

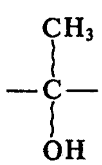

dont le radical —OH peut avoir la configuration *α* ou la configuration *β*,

D représente un radical alkylène, linéaire ou ramifié, contenant de 1 à 5 atomes de carbone, et

$R_4$ représente un radical alkyle contenant de 1 à 7 atomes de carbone,

et également, dans le cas où $R_2$ représente un atome d'hydrogène, les sels que forment ces composés avec des bases acceptables du point de vue physiologique, procédé caractérisé en ce qu'on introduit, de

manière connue, une double liaison en position 2 dans un composé de formule générale II:

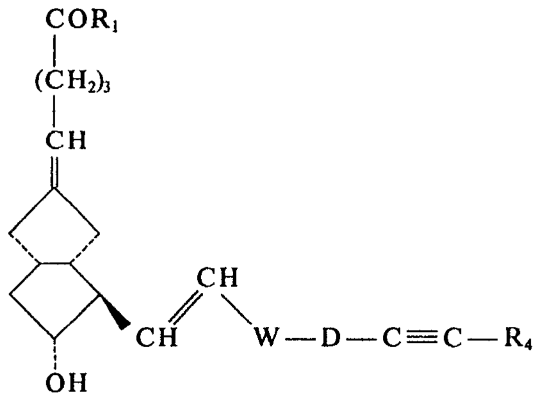

(II)

cela en faisant réagir un dialkylamidure de lithium répondant à la formule générale III:

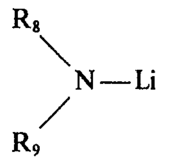

(III)

dans laquelle $R_8$ et $R_9$ représentent chacun un alkyle contenant de 1 à 10 atomes de carbone, un cycloalkyle contenant de 3 à 6 atomes de carbone ou un trialkylsilyle dont chacun des alkyles contient de 1 à 4 atomes de carbone, en traitant par un composé phénylique répondant à la formule générale IV:

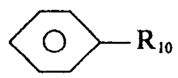

(IV)

dans laquelle $R_{10}$ représente un radical

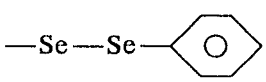 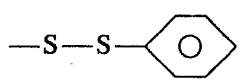 ou —Se—Br

et en oxydant, et ensuite, le cas échéant, on effectue dans l'ordre que l'on veut les opérations suivantes: on sépare des isomères et/ou on libère des radicaux hydroxy protégés et/ou estérifie ou éthérifie des radicaux hydroxy libres et/ou on estérifie un radical carboxy libre et/ou on saponifie un radical carboxy estérifié ou on transforme un radical carboxy en un radical $-CONHR_3$ ou, au moyen d'une base acceptable du point de vue physiologique, en un sel.